(19) Europäisches Patentamt
European Patent Office
Office européen des brevets

(11) EP 0 902 024 B1

# (12) FASCICULE DE BREVET EUROPEEN

(45) Date de publication et mention de la délivrance du brevet:
**19.03.2003 Bulletin 2003/12**

(51) Int Cl.[7]: **C07D 321/10**, C11B 9/00

(21) Numéro de dépôt: **98115434.7**

(22) Date de dépôt: **17.08.1998**

(54) **7-propyl-benzodioxépin-3-one et son utilisation en parfumerie**

7-propyl-benzodiosepin-3-one und dessen Verwendung in Parfümerie

7-propyl-benzodioxepin-3-one and its use in perfumery

(84) Etats contractants désignés:
**CH DE ES FR GB IT LI NL**

(30) Priorité: **09.09.1997 CH 212397**

(43) Date de publication de la demande:
**17.03.1999 Bulletin 1999/11**

(73) Titulaire: **FIRMENICH SA**
**1211 Genève 8 (CH)**

(72) Inventeurs:
- **Gaudin, Jean-Marc**
  **74100 Annemasse (FR)**
- **Blanc, Pierre-Alain**
  **1263 Crassier (CH)**

(74) Mandataire:
**Salvaterra-Garcia, Maria de Lurdes, Dr.**
**Firmenich SA**
**Case Postale 239**
**1211 Genève 8 (CH)**

(56) Documents cités:
**US-A- 3 584 002** **US-A- 3 799 892**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 902 024 B1

## Description

La présente invention a trait au domaine de la parfumerie. Elle concerne plus particulièrement la 7-propyl-2H,4H-1,5-benzodioxépin-3-one et son utilisation en tant qu'ingrédient parfumant.

On connaît du brevet US3584002 et du brevet US 3,799,892 une famille de composés apparentée à la benzodioxepinone de l'invention. Parmi le grand nombre de composés décrits dans ce deuxième document, on cite notamment les composés de formule

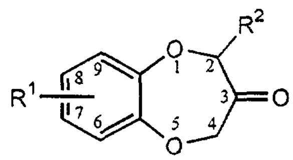

(A)

dans laquelle $R^1$ et $R^2$ représentent l'hydrogène ou un groupe alkyle ayant de 1 à 4 atomes de carbone, et plus particulièrement les composés de formule (A) possédant un substituant méthyle en position 7 ou 8 ($R^2$=H) ou deux substituants méthyles en positions 2 et 7 de la structure moléculaire susmentionnée. Malgré la description d'une quinzaine de composés de formule (A) dans US 3,799,892, ce document ne fait aucune référence à la 7-propyl-2H,4H-1,5-benzodioxépin-3-one qui fait l'objet de la présente invention. Plus curieux encore est le fait que, même si l'objet de l'étude de l'art antérieur portait aussi bien sur des benzoxepinones, que sur des benzodioxepinones, et que chacune de ces espèces chimiques pouvait être substituée par un grand nombre et variété de groupes alkyles, les auteurs affirment que toutes les cétones y-décrites, qu'ils dénomment génériquement "cétones pastèque", confèrent aux parfums, eaux de toilette et cosmétiques dans lesquels elles sont incorporées, des odeurs fraîches, de type feuilles, réminiscentes de l'odeur du melon, leur effet parfumant susmentionné se révélant particulièrement heureux dans des compositions à caractère vert ou feuilles de violette.

A notre grande surprise, il apparaît clairement que les auteurs de cette étude ne connaissaient pas la 7-propyl-2H,4H-1,5-benzodioxépin-3-one de la présente invention, ni ses propriétés particulières, qui rendent son utilisation d'une grande importance dans l'industrie de la parfumerie, et notamment pour le parfumage de produits fonctionnels. En effet, il est bien connu que, par exemple, les milieux des compositions détergentes sont généralement très agressifs envers certaines matières premières de parfumerie dont les notes odorantes sont pourtant particulièrement appréciées pour ce type d'application. L'un des cas les plus flagrants est celui des aldéhydes insaturés d'usage courant, tels l'aldéhyde C11, l'aldéhyde Intreleven (origine International Flavors & Fragrances, Inc.), le 2,6,10-triméthyl-9-undécénal et autres, qui se révèlent totalement instables dans beaucoup de formulations actuelles de détergents textiles contenant des agents et activateurs de blanchissage tels que la tétraacétyléthylènediamine (TAED), des hypohalites, hypochlorites en particulier, des agents de blanchissage péroxygénés, etc.. Les qualités olfactives de ces aldéhydes, qui sont typiquement utilisés pour leur capacité à conférer des notes couramment associées à la perception de l'odeur de linge propre, font que leur utilisation est malgré tout très répandue, mais leur instabilité dans ces milieux reste un problème d'actualité, que l'on cherche à résoudre par l'emploi de matières alternatives d'un point de vue odorant.

Or, la présente invention apporte justement une solution nouvelle et avantageuse au problème susmentionné. Nous avons en effet établi que la 7-propyl-2H,4H-1,5- benzodioxépin-3-one développe une odeur fortement aldéhydée, très puissante et réminiscente de l'odeur du 2,6,10-triméthyl-9-undécénal en particulier, accompagnée cependant d'une connotation épicée et d'un caractère qui évoque l'odeur du linge propre séchant au soleil.

L'odeur de ce composé est, par ailleurs, totalement distincte de celle de ses analogues et homologues décrits dans le brevet mentionné plus haut et dont la 7-méthyl-2H,4H-1,5-benzodioxépin-3-one est, à notre connaissance, le seul produit de la série décrite qui a fait l'objet d'une exploitation commerciale (produit commercialisé sous le nom de CALONE® ; origine : C.A.L. SA, Grasse, France).

Or, la Calone®, par exemple, développe une odeur fleurie-verte, à connotation fortement ozonée, marine, rappelant l'odeur des huîtres. Nous avons par ailleurs pu constater que cette note marine, ozonée est assez typique des composés de la série connue, et présente, à des degrés d'importance variés, dans l'odeur des composés de formule (A) dans laquelle $R^2$ représente l'hydrogène et $R^1$ représente l'hydrogène ou un substituant éthyle, isopropyle, tert-butyle ou 1,1-diméthylpropyle en position 7 du cycle benzénique, alors qu'elle est complètement absente de l'odeur du composé qui fait l'objet de la présente invention. Aucun de ces analogues, qui sont les plus proches structurellement de la 7-propyl-2H,4H-1,5-benzodioxépin-3-one de l'invention, ne possède par ailleurs le caractère fortement aldéhydé, épicé qui donne toute la valeur parfumistique à cette dernière dans le contexte de l'invention, la rendant un ingrédient de choix pour le parfumage de compositions détergentes à usages multiples, ainsi que d'adoucissants textiles, dans lesquels la puissance et substantivité de son odeur apporte un atout supplémentaire. C'est ainsi que nous avons pu constater que, par exemple, les textiles lavés en présence de détergents ou adoucissants textiles contenant la 7-propyl-2H,4H-1,5-benzodioxépin-3-one exhalent une odeur de linge propre et frais qui est très durable et qui devient particulièrement remarquée lors du repassage des vêtements, même lorsque celui-ci a lieu plusieurs jours après leur séchage.

Ainsi, l'invention concerne également l'emploi préféré du composé susmentionné dans des applications telles que savons, shampoings, détergents liquides ou solides destinés au traitement de textiles, adoucissants textiles, ou encore des compositions détergentes ou produits d'entretien destinés au nettoyage de la vaisselle ou de surfaces variées, qu'ils soient voués à un usage domestique ou industriel.

D'une façon générale, la 7-propyl-2H,4H-1,5-benzodioxépin-3-one se révèle être un ingrédient parfumant dont l'utilisation est à son mieux dans tous les produits de consommation où les substances parfumantes ayant des fonctions aldéhydiques dans leur structure sont chimiquement instables. On peut citer dans ce contexte, parmi d'autres, tous les milieux fortement réducteurs ou oxydants, notamment les détergents et produits de blanchissage, contenant des agents ou activateurs de blanchissage ou des produits chlorés, ainsi que les désodorisants et antiperspirants corporels qui contiennent par exemple des sels d'aluminium. Comme il ressort des exemples présentés plus loin, la benzodioxepinone de l'invention se révèle parfaitement stable dans ces milieux, alors que sa note odorante typiquement aldéhydée se prête particulièrement bien aux fragrances usuelles de ces produits de consommation.

La présente invention met ainsi à la disposition du parfumeur un produit unique à connotation typiquement aldéhydée, pouvant être librement utilisé dans les milieux où l'usage des produits odorants courants susceptibles de conférer cet effet parfumant lui est souvent interdit, ou en tout cas rendu très difficile.

Bien entendu, son utilisation n'est cependant pas limitée aux produits mentionnés plus haut, et ce composé se prête également à tous les autres emplois courants en parfumerie, à savoir la préparation de parfums et eaux de toilette, et au parfumage de savons et gels de bain ou douche, produits d'hygiène ou traitement des cheveux, ainsi que désodorisants ambiants ou encore préparations cosmétiques, et autres produits de soins de cheveux.

Dans ces applications, il peut être utilisé seul ou en mélange avec d'autres ingrédients parfumants, des solvants ou adjuvants d'usage courant en parfumerie. La nature et variété de ces coingrédients n'appelle pas une description plus détaillée ici, qui ne saurait d'ailleurs être exhaustive, l'homme de l'art étant à même de les choisir de par ses connaissances générales et en fonction de la nature du produit à parfumer et de l'effet olfactif recherché. Ces ingrédients parfumants appartiennent à des classes chimiques aussi variées que les alcools, aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres oeuvres de nature similaire.

Les proportions dans lesquelles le composé selon l'invention peut être incorporé dans les produits divers susmentionnés varient dans une gamme de valeurs étendue. Ces valeurs sont dépendantes de la nature de l'article ou produit que l'on veut parfumer et de l'effet olfactif recherché, ainsi que de la nature des coingrédients dans une composition donnée lorsque le composé de l'invention est utilisé en mélange avec des coingrédients parfumants, des solvants ou des adjuvants d'usage courant dans l'art.

A titre d'exemple, on peut citer des concentrations typiques de l'ordre de 0,1 à 1%, voire plus, en poids de ce composé, par rapport au poids de composition parfumante dans laquelle il est incorporé. Des concentrations bien inférieures à celles-ci peuvent être utilisées lorsque ce composé est directement appliqué dans le parfumage des produits de consommation divers cités auparavant.

L'invention sera maintenant décrite de façon plus détaillée dans les exemples suivants.

Exemple 1

Préparation de 7-propyl-2H,4H-1,5-benzodioxépin-3-one

Ce composé a été préparé à l'aide de réactions connues, à partir du 4-propyl-1,2-benzènediol, pur à 97%. Ce produit de départ, à odeur cuivrée, phénolique, légèrement grasse, a été transformé de façon analogue à celle décrite dans US 3,799,892 pour fournir un produit brut qui, après purification et cristallisation dans l'heptane à -20°C a fourni la 7-propyl-2H,4H-1,5-benzodioxépin-3-one avec une pureté supérieure à 99% et les caractéristiques spectrales suivantes :

RMN($^{13}$C): 13,75(q); 24,42(t); 37,09(t); 75,49(t); 75,77(t); 120,53(d); 120,60(d); 123,71(d); 138,71(s); 146,22(s); 147,98(s); 204,85(s) δ ppm

RMN($^{1}$H) : 0,93(t, J=8, 3H) ; 1,60(m, 2H) ; 2,50(t, J=6, 2H); 4,66(s, 2H) ; 4,70(s, 2H) ; 6,76(dd, J=2, J=8, 1H) ; 6,80 (d, J=2, 1H); 6,90(d, J=8, 1H) δ ppm

SM : 206($M^+$) : 177(100), 149(30), 135(10), 123(8), 105(5), 91(10), 77(18), 65(10), 55(8), 51(10), 39(10).

Exemple 2

Préparation d'une composition parfumante pour un savon

On a préparé une composition parfumante de base à caractère fleuri-poudré, destinée à un savon, en mélangeant les ingrédients suivants :

| Ingrédient | Parties en poids |
|---|---|
| Acétate de benzyle | 80 |
| Acétate de citronellyle | 50 |
| Acétate de verdyle | 80 |
| Aldéhyde anisique | 30 |
| Undécanal à 50%* | 20 |
| Cétalox ® 1) à 10%* | 20 |
| Aldéhyde cyclamen | 15 |
| Dihydromyrcénol 2) | 40 |
| Habanolide ® 3) | 45 |
| Hédione ® 4) | 50 |
| Iralia ® 5) | 40 |
| Iso E Super 6) | 80 |
| Lilial ® 7) | 60 |
| Lorysia ® 8) | 220 |
| Méthyl octyne carbonate à 10%* | 10 |
| Polysantol ® 9) | 10 |
| Salicylate d'hexyle | 150 |
| Total | 1000 |

* dans le dipropylèneglycol (DIPG)
1) dodécahydro-3a,6,6,9a-tétraméthyl-naphtho[2,1-b]furanne ; origine : Firmenich SA, Genève, Suisse
2) 2,6-diméthyl-7-octén-2-ol ; origine : International Flavors & Fragrances, USA
3) pentadécénolide ; origine : Firmenich SA, Genève, Suisse
4) dihydrojasmonate de méthyle ; origine : Firmenich SA, Genève, Suisse
5) méthylionone ; origine : Firmenich SA, Genève, Suisse
6) origine : International Flavors & Fragrances, USA
7) 2-méthyl-3-(4-tert-butyl-1-phényl)-propanal; origine : Givaudan-Roure, Vernier, Suisse
8) acétate de 4-(1,1-diméthyléthyl)-cyclohexanol; origine : Firmenich SA, Genève, Suisse
9) 3,3-diméthyl-5-(2,2,3-triméthyl-3-cyclopenten-1-yl)-4-pentén-2-ol ; origine Firmenich SA, Genève, Suisse

L'addition de 1% en poids de 7-propyl-2H,4H-1,5-benzodioxépin-3-one à cette composition de base lui a conféré une note aldéhydée-métallique très puissante, qui rappelait un peu l'effet odorant que l'on peut obtenir à l'aide du 2,6,10-triméthyl- 9-undécénal ou de l'aldéhyde 2-méthylundécanal, mais accompagné en plus d'une jolie note florale rappelant l'odeur de l'héliopropanal (origine : International Flavors & Fragrances, USA), et d'une sous-note épicée très agréable. Par ailleurs, la fragrance de la composition s'en est trouvée nettement améliorée et son impact grandement renforcé lorsque cette composition nouvelle a été ajoutée à un savon, à une concentration de 1% en poids de composition par rapport au poids du savon.

Exemple 3

Préparation d'une composition parfumante

On a préparé une eau de toilette de base, à odeur fleurie, boisée, herbacée, en mélangeant les ingrédients suivants :

| Ingrédient | Parties en poids |
|---|---|
| Essence d'absinthe | 200 |
| Acétate de linalyle | 400 |

(suite)

| Ingrédient | Parties en poids |
|---|---|
| Aldéhyde Intreleven [1)] à 10%* | 10 |
| Glycolate d'allyle amyle | 100 |
| Essence d'armoise | 70 |
| Essence de cannelle de Ceylan | 10 |
| Essence de cardamome | 50 |
| Coumarine | 30 |
| α-Damascone | 20 |
| Dihydromyrcénol[2)] | 900 |
| Estragole | 40 |
| Floropal ® [3)] | 50 |
| Essence de galbanum à 10%* | 40 |
| Essence de girofle | 80 |
| Habanolide ® [2)] | 330 |
| Hédione ® [2)] | 300 |
| Iralia ®[2)] | 80 |
| Isobutylquinoléine [4)] | 20 |
| Essence de lavandin | 150 |
| Essence de mandarine | 140 |
| Mousse cristal | 100 |
| Oxyde de rose à 10%* | 40 |
| Essence de patchouli | 280 |
| Polysantol ® [2)] | 100 |
| Essence de sauge sclarée | 100 |
| Triplai ® [5)] à 10%* | 120 |
| Galbex ® [6)] 183 | 180 |
| Total | 3990 |

* dans le DIPG
1) undécénal ; origine : International Flavors & Fragrances, USA
2) voir Exemple 2
3) 2,4,6-triméthyl-4-phényl-1,3-dioxane ; origine : Allemagne Haarman & Reimer GmbH,
4) origine : International Flavors & Fragrances, USA
5) 2,4-diméthyl-3-cyclohexène-1-carboxaldéhyde; Fragrances, USA origine : International Flavors &
6) origine : Firmenich SA, Genève, Suisse

L'addition de 10 parties en poids de 7-propyl-2H,4H-1,5-benzodioxépin-3-one à cette composition de base lui a conféré une connotation moderne, plus fraîche, plus épicée, tout en accroissant son impact odorant, et en lui donnant un caractère herbacé plus naturel entièrement dû à l'effet odorant aldéhydique typique du composé de l'invention. Ce dernier a par ailleurs totalement étouffé la note terreuse, sale, due à l'essence de patchouli.

Exemple 4

Préparation d'une composition parfumante pour un détergent

On a préparé une composition parfumante de base, destinée au parfumage d'un détergent, à l'aide des ingrédients suivants :

| Ingrédient | Parties en poids |
|---|---|
| Aldéhyde hexylcinnamique | 200 |
| Cétalox ® [1)] | 15 |

1) voir Exemple 2

(suite)

| Ingrédient | Parties en poids |
|---|---|
| cis-3-Hexénol à 10%* | 20 |
| Citronellyl nitrile | 5 |
| Habanolide ® [1] | 80 |
| Hédione ® [1] | 70 |
| Indol à 10%* | 5 |
| Iralia ® [1] Total | 70 |
| Iso E Super [1] | 70 |
| Linalol | 80 |
| Lorysia ® [1] | 120 |
| Manzanate ® [2] à 10%* | 20 |
| Mayol ® [3] | 60 |
| trans-1-(2,2,6-Triméthyl-1-cyclohexyl)-3-hexanol [4] à 10%* | 20 |
| 3-Méthyl-5-phényl-1-pentanol [4] | 80 |
| Polysantol ® [1] | 15 |
| Vertofix Coeur [5] | 60 |
| Total | 980 |

* dans le DIPG
1) voir Exemple 2
2) 2-méthyl-pentanoate d'éthyle ; origine : Quest International Fragrances
3) 7-p-menthanol ; origine Firmenich SA, Genève, Suisse
4) origine : Firmenich SA, Genève, Suisse
5) origine : International Flavors & Fragrances, USA

Lorsqu'on a ajouté à cette composition de base à caractère floral, poudré, boisé, 0,2% en poids de 7-propyl-2H,4H-1,5-benzodioxépin-3-one, on a obtenu une composition nouvelle dont l'odeur possédait le caractère frais, linge propre que l'on ne pouvait obtenir jusqu'ici qu'à l'aide des aldéhydes aliphatiques insaturés (aldéhyde C11, aldéhyde Intreleven, etc) typiquement utilisés en parfumerie classique pour cet effet. Le parfum ainsi obtenu convient à toute utilisation dans des détergents courants, notamment ceux contenant la TAED, dans lesquels ces aldéhydes s'avèrent être totalement instables.

Exemples 5 à 20

On a procédé au parfumage des articles et produits mentionnés ci-après, en ajoutant la 7-propyl-2H,4H-benzodioxépin-3-one, dans les concentrations indiquées, aux bases appropriées non parfumées :

| | Produit | Concentration (% en poids) | Odeur */ Aspect | | | Puissance de l'odeur et couverture de la base |
|---|---|---|---|---|---|---|
| | | | [3°C] | [22°C] | [40°C] | |
| 5 | Eau de toilette alcoolique (alcool 95°) | 5,0 | S/N | S/N | S/N | TP / CN |
| 6 | Crème huile/eau | 0,5 | S/N | S/N | S/N | TP / CN |
| 7 | Crème eau/huile | 0,5 | S/N | S/N | S/N | P / CN |
| 8 | Shampoing | 0,5 | S/N | S/N | S/N | P / CN |
| 9 | Après-shampoing | 0,3 | S/N | S/N | S/N | TP / CN |

* S - stable = l'odeur du produit parfumé n'a pas été modifiée par rapport au composé
OM - odeur modifiée
N - normal = l'aspect du produit parfumé n'a pas changé par rapport à la base
LC - légère coloration
TP - odeur très puissante
P - odeur puissante
CN - l'odeur du composé parfumant couvre normalement celle de la base

(suite)

| | Produit | Concentration (% en poids) | Odeur */ Aspect | | | Puissance de l'odeur et couverture de la base |
|---|---|---|---|---|---|---|
| | | | [3°C] | [22°C] | [40°C] | |
| 10 | Talc | 0,5 | S/N | S/N | S/N | TP / CN |
| 11 | Antiperspirant (roll-on) | 0,5 | S/N | S/N | S/N | TP / CN |
| 12 | Antiperspirant (spray) | 1,0 | S/N | S/N | S/LC | P / CN |
| 13 | Désodorisant (spray) | 1,3 | S/N | S/N | S/N | TP / CN |
| 14 | Spray laque | 0,4 | S/N | S/N | S/N | TP / CN |
| 15 | Savon | 1,0 | S/N | S/N | S/LC | P / CN |
| 16 | Détergent en poudre contenant des perborates | 0,2 | S | S | S | TP / CN |
| 17 | Détergent en poudre conc. contenant TAED | 0,2 | S | S | S | TP / CN |
| 18 | Adoucissant textile | 0,2 | S | S | OM | P / CN |
| 19 | Eau de Javel | 0,2 | S | S | OM | P / CN |
| 20 | Acide HCl à 5% | 0,2 | S | S | S | TP / CN |

* S - stable = l'odeur du produit parfumé n'a pas été modifiée par rapport au composé
OM - odeur modifiée
N - normal = l'aspect du produit parfumé n'a pas changé par rapport à la base
LC - légère coloration
TP - odeur très puissante
P - odeur puissante
CN - l'odeur du composé parfumant couvre normalement celle de la base

Les résultats des essais présentés au Tableau montrent que la 7-propyl-2H,4H-1,5-benzodioxépin-3-one est parfaitement stable dans une grande variété de produits de consommation et dans les conditions normales de leur utilisation ou stockage, et couvre efficacement l'odeur de la base là où il y a lieu.

## Revendications

1. 7-Propyl-2H,4H-1,5-benzodioxépin-3-one.

2. Composition parfumante ou produit parfumé contenant la 7-propyl-2H,4H-1,5-benzodioxépin-3-one en tant qu'ingrédient parfumant.

3. Produit parfumé selon la revendication 2, sous forme d'un parfum ou d'une eau de toilette, d'une préparation cosmétique, ou d'un shampoing ou autre produit de soins des cheveux, d'un savon ou d'un gel de bain ou douche, ou d'un désodorisant ambiant.

4. Désodorisant ou antiperspirant corporel, contenant à titre d'ingrédient parfumant la 7-propyl-2H,4H-1,5-benzodioxépin-3-one.

5. Détergent ou adoucissant textile, contenant la 7-propyl-2H,4H-1,5-benzodioxépin-3-one à titre d'ingrédient parfumant.

6. Composition détergente à usage domestique ou industriel, contenant la 7-propyl-2H,4H-1,5-benzodioxépin-3-one à titre d'ingrédient parfumant.

7. Composition détergente selon la revendication 6, sous forme d'un détergent à vaisselle ou d'un produit d'entretien de surfaces dures.

8. Utilisation de la 7-propyl-2H,4H-1,5-benzodioxépin-3-one pour le parfumage de produits de consommation contenant des substances chlorées, des agents ou activateurs de blanchissage, des agents réducteurs ou oxydants.

## Claims

1. 7-Propyl-2H,4H-1,5-benzodioxepin-3-one.

2. Perfuming composition or perfumed product containing 7-propyl- 2H,4H-1,5-benzodioxepin-3-one as perfuming ingredient.

3. Perfumed product according to claim 2, in the form of a perfume or a cologne, a cosmetic preparation, or a shampoo or other hair-care product, a soap or a bath or shower gel, or an air freshener.

4. Deodorant or antiperspirant, containing 7-propyl-2H,4H-1,5-benzodioxepin-3-one as perfuming ingredient.

5. Detergent or fabric softener, containing 7-propyl-2H,4H-1,5-benzodioxepin-3-one as perfuming ingredient.

6. Detergent composition for household or industrial use, containing 7-propyl-2H,4H-1,5-benzodioxepin-3-one as perfuming ingredient.

7. Detergent composition according to claim 6, in the form of a dishwashing detergent or a cleaning product for hard surfaces.

8. Use of 7-propyl-2H,4H-1,5-benzodioxepin-3-one for perfuming consumer products containing chlorinated substances, bleaching agents or activators, reducing or oxidizing agents.

## Patentansprüche

1. 7-Propyl-2H,4H-1,5-benzodioxepin-3-on.

2. Duftzusammensetzung oder wohlriechendes Produkt, enthaltend 7-Propyl-2H,4H-1,5-benzodioxepin-3-on als Duftstoff.

3. Wohlriechendes Produkt nach Anspruch 2 in Form eines Parfüms oder eines Eau de Toilette, eines Kosmetikpräparats, oder eines Shampoos oder eines anderen Haarpflegeprodukts, einer Seife oder eines Bade- oder Duschgels, oder eines Raumdeodorants.

4. Körperdeodorant oder -antitranspirant, enthaltend als Duftstoff 7-Propyl-2H,4H-1,5-benzodioxepin-3-on.

5. Textilwaschmittel oder -weichspüler, enthaltend 7-Propyl-2H,4H-1,5-benzodioxepin-3-on als Duftstoff.

6. Haushalts- oder Industriereiniger, enthaltend 7-Propyl-2H,4H-1,5-benzodioxepin-3-on als Duftstoff.

7. Reiniger nach Anspruch 6 in Form eines Geschirrspülmittels oder eines Pflegeprodukts für harte Oberflächen.

8. Verwendung von 7-Propyl-2H,4H-1,5-benzodioxepin-3-on zur Parfümierung von Verbrauchsartikeln, die chlorhaltige Substanzen, Bleichmittel oder Bleichaktivatoren, Reduktions- oder Oxidationsmittel enthalten.